(19) Europäisches Patentamt
European Patent Office
Office européen des brevets

(11) Publication number: **0 244 364**
**A2**

(12) # EUROPEAN PATENT APPLICATION

(21) Application number: 87810277.1

(22) Date of filing: 30.04.87

(51) Int. Cl.[3]: C 07 D 209/18
C 07 D 233/26, C 07 C 59/48
C 07 F 7/18, C 07 C 27/02
C 07 B 53/00
//C07C69/38, C07F9/65, C07F9/40, C07C69/675, C07C69/708, C07C59/13, C07C69/716

(30) Priority: 30.04.86 US 857689

(43) Date of publication of application: 04.11.87 Bulletin 87/45

(84) Designated Contracting States: AT BE CH DE ES FR GB GR IT LI LU NL SE

(71) Applicant: SANDOZ AG
Lichtstrasse 35
CH-4002 Basel(CH)

(84) Designated Contracting States: BE CH ES FR GB GR IT LI LU NL SE

(71) Applicant: SANDOZ-PATENT-GMBH
Humboldtstrasse 3
D-7850 Lörrach(DE)

(84) Designated Contracting States: DE

(71) Applicant: SANDOZ-ERFINDUNGEN Verwaltungsgesellschaft m.b.H.
Brunner Strasse 59
A-1235 Wien(AT)

(84) Designated Contracting States: AT

(72) Inventor: Chen, Kau-Ming
5 Tammy Hill Trail
Randolph, N.J. 07869(US)

(72) Inventor: Hardtmann, Goetz Edward
12 Lynnfield Drive
Morristown N.J. 07960(US)

(72) Inventor: Lee, George T.
16 Macleod Lane
Bloomfield, N.J. 07003(US)

(72) Inventor: Linder, Jerome
722 Crescent Parkway
Westfield, N.J. 07090(US)

(72) Inventor: Wattanasin, Sompong
30 Beech Street
Stanhope, N.J. 07874(US)

(72) Inventor: Kapa, Prasad Koteswara
4 Faber Road
Parsippany, N.J. 07054(US)

(54) **Preparation of olefinic compounds.**

(57) A process for preparing 7-substituted 3,5-dihydroxy-hept-6-enoic acid derivatives in substantially pure 3R,5S-form employing as starting material L-malic acid.

EP 0 244 364 A2

# PREPARATION OF OLEFINIC COMPOUNDS

This invention relates to a process for preparing organic compounds, and more specifically for preparing 7-substituted 3,5-dihydroxy-hept-6-enoic acid derivatives, as well as intermediates, *per se* in the process.

This invention provides a novel process for the preparation of *trans*-olefins of the formula I:

I 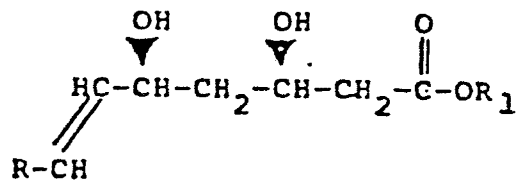

wherein $R_1$ is hydrogen, $R_2$; or M, wherein $R_2$ is a physiologically acceptable and hydrolyzable ester group, and M is a pharmaceutically acceptable cation; and R is any of radicals A to H, whereby in each case X stands for

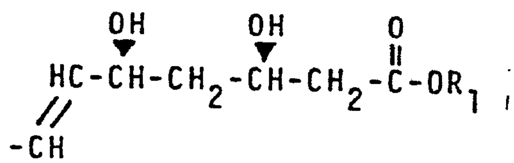

A) 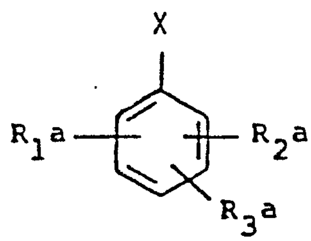

in which each of the $R_1a$, $R_2a$ and $R_3a$ is independently hydrogen; halogen; $C_{1-4}$alkyl; $C_{1-4}$haloalkyl; phenyl, or phenyl substituted by halogen, $C_{1-4}$alkoxy, $C_{2-8}$alkanoyloxy, $C_{1-4}$alkyl or $C_{1-4}$haloalkyl, or $OR^d$ in which $R^d$ is any of hydrogen, $C_{2-8}$alkanoyl, benzoyl, phenyl, halophenyl, phenyl $C_{1-3}$alkyl, $C_{1-9}$alkyl, cinnamyl, $C_{1-4}$haloalkyl,

allyl, cycloalkyl-$C_{1-3}$alkyl, adamantyl-$C_{1-3}$alkyl, or substituted phenyl-$C_{1-3}$alkyl in each of which the substituents are selected from the group consisting of halogen, $C_{1-4}$alkoxy, $C_{1-4}$alkyl, or $C_{1-4}$haloalkyl (the halogen atoms including fluoro or chloro, and cycloalkyl including cyclohexyl); a naphthyl or tetrahydronaphthyl structure of formula B:

B 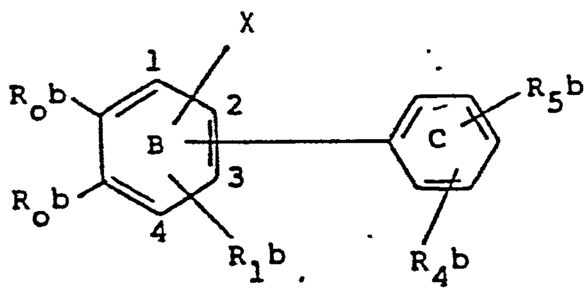

wherein the two $R_o^b$ groups together form a radical of the formula

a) 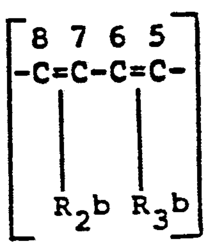 or b) $-CH_2CH_2CH_2CH_2-$,

wherein $R_2b$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy;

$R_3b$ is hydrogen, $C_{1-3}$alkyl, $C_{1-3}$alkoxy, trifluoromethyl, fluoro, chloro, phenoxy or benzyloxy, with the provisos that not more than one of $R_2^b$ and $R_3^b$ is trifluoromethyl, not more than one of $R_2^b$ and $R_3^b$ is phenoxy, and not more than one of $R_2^b$ and $R_3^b$ is benzyloxy;

$R_1b$ is hydrogen, $C_{1-3}$alkyl, fluoro, chloro or benzyloxy;

$R_4b$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy;

$R_5b$ is hydrogen, $C_{1-3}$alkyl, $C_{1-3}$alkoxy, trifluoromethyl, fluoro, chloro, phenoxy or benzyloxy, with the provisos that not more than one of $R_4^b$ and $R_5^b$ is trifluoromethyl, not more than one of $R_4b$ and $R_5b$ is phenoxy, and not more than one of $R_4b$ and $R_5b$ is benzyloxy;

optionally Ring C may also bear a $C_{1-2}$alkyl, $C_{1-2}$alkoxy, fluoro or chloro;

with the proviso that the side chain X and the $R_4b$-bearing the phenyl group (ring C) are ortho to each other;

an indolyl structure of the formula C:

C

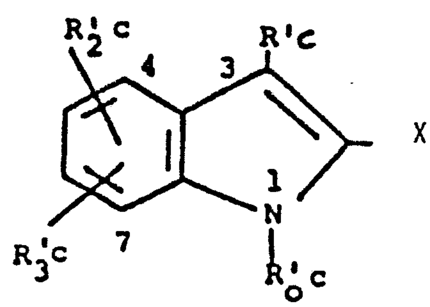

wherein one of R'c and $R_o'c$ is 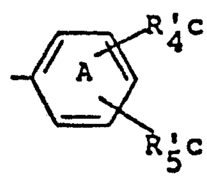 and the other is

$C_{1-3}$alkyl, n-butyl or i-butyl,

wherein $R_4'c$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy; and

$R_5'c$ is hydrogen, $C_{1-3}$alkyl, $C_{1-3}$alkoxy, trifluoromethyl, fluoro, chloro, phenoxy or benzyloxy, with the proviso that not more than one of $R_4'c$ and $R_5'c$ is trifluoromethyl, not more than one of $R_4'c$ and $R_5'c$ is phenoxy and not more than one of $R_4'c$ and $R_5'c$ is benzyloxy;

$R_2'c$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy;

$R_3'c$ is hydrogen, $C_{1-3}$alkyl, $C_{1-3}$alkoxy, trifluoromethyl, fluoro, chloro, phenoxy or benzyloxy ;

optionally Ring A may also bear a $C_{1-2}$alkyl, $C_{1-2}$alkoxy, fluoro or chloro;

with the provisos that not more than one of $R_2'c$ and $R_3'c$ is trifluoromethyl, not more than one of $R_2'c$ and $R_3'c$ is phenoxy, and not more than one of $R_2'c$ and $R_3'c$ is benzyloxy;

an imidazole radical of the formula D:

D 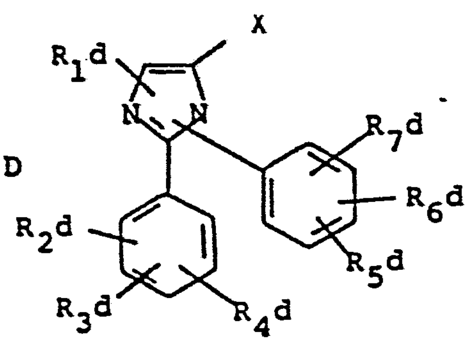

wherein $R_1d$ is $C_{1-6}$alkyl not containing an asymmetric carbon atom;

each of $R_2d$ and $R_5d$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, phenyl, phenoxy or benzyloxy;

each of $R_3d$ and $R_6d$ is independently hydrogen, $C_{1-3}$alkyl, $C_{1-3}$alkoxy, trifluoromethyl, fluoro, chloro, phenoxy or benzyloxy;

each of $R_4d$ and $R_7d$ is independently hydrogen, $C_{1-2}$alkyl, $C_{1-2}$alkoxy, fluoro or chloro;

with the provisos that not more than one of $R_2d$ and $R_3d$ is trifluoromethyl, not more than one of $R_2d$ and $R_3d$ is phenoxy, not more than one of $R_2d$ and $R_3d$ is benzyloxy, not more than one of $R_5d$ and $R_6d$ is trifluoromethyl, not more than one of $R_5d$ and $R_6d$ is phenoxy, and not more than one of $R_5d$ and $R_6d$ is benzyloxy;

an indenyl radical of the formula E:

E 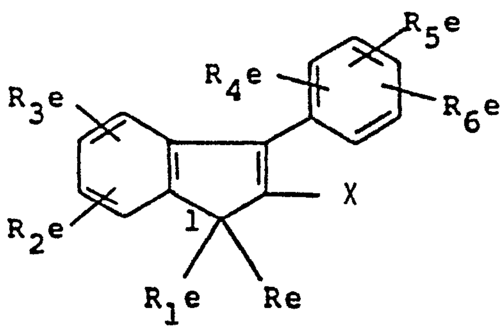

wherein Re is hydrogen or primary or secondary $C_{1-6}$alkyl not containing an asymmetric carbon atom, and

$R_1e$ is primary or secondary $C_{1-6}$alkyl not containing an asymmetric carbon atom or

Re and $R_1e$ taken together are $-(CH_2)_m-$ or $(Z)-CH_2-CH=CH-CH_2-$, wherein m is 2, 3, 4, 5 or 6;

$R_2e$ is hydrogen, $C_{1-3}$alkyl, n-butyl, i-butyl, t-butyl, $C_{1-3}$alkoxy, n-butoxy, i-butoxy, trifluoromethyl, fluoro, chloro, phenoxy or benzyloxy;

$R_3e$ is hydrogen, $C_{1-3}$alkyl, $C_{1-3}$alkoxy, trifluoromethyl, fluoro, chloro, phenoxy or benzyloxy, with the provisos that not more than one of $R_2e$ and $R_3e$ is trifluoromethyl, not more than one of $R_2e$ and $R_3e$ is phenoxy, and not more than one of $R_2e$ and $R_3e$ is benzyloxy;

$R_4e$ is hydrogen, $C_{1-3}$alkyl, n-butyl, i-butyl, t-butyl, $C_{1-3}$alkoxy, n-butoxy, i-butoxy, trifluoromethyl, fluoro, chloro, phenoxy or benzyloxy;

$R_5e$ is hydrogen, $C_{1-3}$alkyl, $C_{1-3}$alkoxy, trifluoromethyl, fluoro, chloro, phenoxy or benzyloxy;

$R_6e$ is hydrogen, $C_{1-2}$alkyl, $C_{1-2}$alkoxy, fluoro or chloro;

with the provisos that not more than one of $R_4e$ and $R_5e$ is trifluoromethyl, not more than one of $R_4e$ and $R_5e$ is phenoxy, and not more than one of $R_4e$ and $R_5e$ is benzyloxy; or

an aryl or partially reduced aryl hydrocarbyl radical of the formula F, wherein F is any of the radicals:

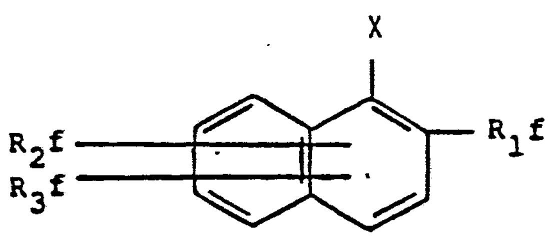

a)

F)

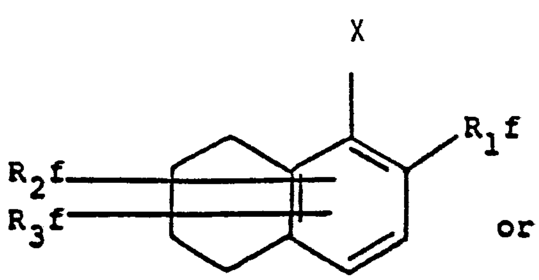

or

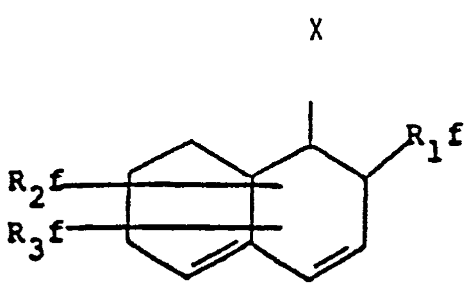

b) c)

in which $R_1f$, $R_2f$ and $R_3f$ are independently, halo having an atomic weight of from about 19 to 35, i.e. F or Cl, hydrogen, or lower alkyl having from 1 to 4 carbon atoms (preferably for radicals a), b) and c) $R_1f$ being methyl);

a pyrazolyl radical of the formula G:

G

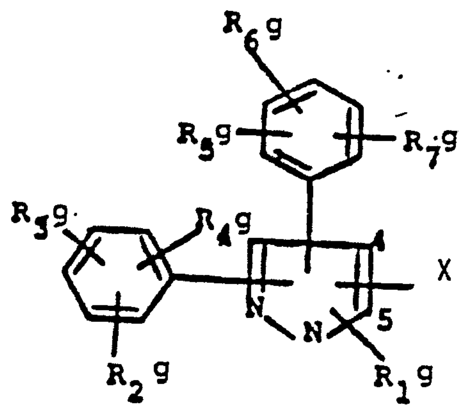

wherein $R_1g$ is $C_{1-6}$alkyl not containing an asymmetric carbon atom;

each of $R_2g$ and $R_5g$ is independently hydrogen, $C_{1-3}$alkyl, n-butyl, i-butyl, t-butyl, $C_{1-3}$alkoxy, n-butoxy, i-butoxy, trifluoromethyl, fluoro, chloro, phenyl, phenoxy or benzyloxy;

each of $R_3g$ and $R_6g$ is independently hydrogen, $C_{1-3}$alkyl, $C_{1-3}$alkoxy, trifluoromethyl, fluoro, chloro, phenoxy or benzyloxy;

each of $R_4g$ and $R_7g$ is independently hydrogen, $C_{1-2}$alkyl, $C_{1-2}$alkoxy, fluoro or chloro; with the provisos that not more than one of $R_2g$ and $R_3g$ is trifluoromethyl; not more than one of $R_2g$ and $R_3g$ is phenoxy, not more than one of $R_2g$ and $R_3g$ is benzyloxy, not more than one of $R_5g$ and $R_6g$ is trifluoromethyl, not more than one of $R_5g$ and $R_6g$ is phenoxy, and not more than one of $R_5g$ and $R_6g$ is benzyloxy;

with the proviso that (i) the side chain X is in the 4- or 5-position of the pyrazole ring, and is <u>Ortho</u> to the $R_1g$ group; or a five-membered ring-heterocyclic radical of the formula H:

H 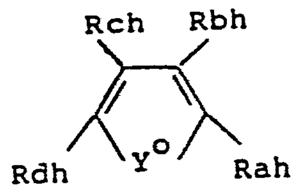

wherein

Rah is X, Rbh is $R_2h$, Rch is $R_3h$, Rdh is $R_4h$ and $Y^o$ is a group -N-; or
$R_1h$

Rah is $R_1h$, Rbh is X, Rch is $R_2h$, Rdh is $R_3h$ and $Y^o$ is O, S or a group -N-;
$R_4h$

$R_1h$, $R_2h$, $R_3h$ and $R_4h$ independently are $C_{1-6}$alkyl not containing an asymmetric carbon atom, $C_{3-7}$cycloalkyl or a ring $A^o$:

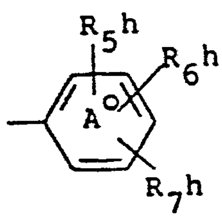 ;

or in the case of $R_3h$ and $R_4h$ additionally hydrogen, or for $R_3h$ when $Y^o$ is O or S 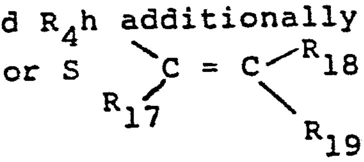 whereby $R_{17}$ is hydrogen or $C_{1-3}$alkyl and $R_{18}$ and $R_{19}$ are independently hydrogen, $C_{1-3}$alkyl or phenyl; each $R_5h$ is independently

hydrogen, $C_{1-3}$alkyl, *n*-butyl, *i*-butyl, *t*-butyl, $C_{1-3}$alkoxy, *n*-butoxy, *i*-butoxy, trifluoromethyl, fluoro, chloro, bromo, phenyl, phenoxy or benzyloxy; each $R_6h$ is independently hydrogen, $C_{1-3}$alkyl, $C_{1-3}$alkoxy, trifluoromethyl, fluoro, chloro, bromo, phenoxy or benzyloxy, and each $R_7h$ is independently hydrogen, $C_{1-2}$alkyl, $C_{1-2}$alkoxy, fluoro or chloro,

with the proviso that there may only be one each of trifluoromethyl, phenoxy or benzyloxy in each ring $A^o$ present.

Compounds I may be viewed as consisting of 3 classes of compounds depending on the definition of $R_1$, ie

Compounds $I_1$ when $R_1 = R_2$;

Compounds $I_2$ when $R_1 = M$; and

Compounds $I_3$ when $R_1 = H$,

$R_1$, $R_2$ and M being as defined above.

Compounds I may be further viewed as consisting of 7 sub-classes depending on the definition of R; ie

Compounds IA when R = A;

Compounds IB when R = B;

Compounds IC when R = C;

Compounds ID when R = D;

Compounds IE when R = E;

Compounds IF when R = F;

Compounds IG when R = G; and

Compounds IH when R = H;

radicals A to H being as defined above.

Final products (I) bearing the above-described forms of the radical R, are disclosed in the art, which also discloses methods of preparation of those radicals, as well as utility for the final products. Compounds I

may be used in the 3,5-dihydroxy-form shown above, or in the form of their corresponding lactones, which may be prepared from them by methods known in the art. In general the final products (I) and their corresponding lactones are useful as anti-atherosclerotic agents as they are active in inhibiting the synthesis of cholesterol in a host by inhibiting the enzyme HMG-CoA reductase.

Compounds in which:

R is of type A) are disclosed in USP 4,375, 475 (issued Mar. 1, 1983);

R is of type B) and disclosed in WO 84/02903 (published Aug. 2, 1984) and U.S. application Serial No. 834,186 (filed Feb. 26, 1986);

R is of type C) are disclosed in WO 84/02131 (published June 7, 1984) and U.S. application Serial No. 722,288 (filed April 11, 1985);

R is of type D) are disclosed in WO 86/07054 (published December 4, 1986) and in U.S. application Serial No. 863,267 (filed May 14, 1986);

R is of type E) are disclosed WO 86/03488 (published June 19, 1986) and in U.S. application Serial No. 837,479 (filed Mar. 7, 1986.);

R is of type F) in the lactone form, are disclosed in U.S. patent 4,474,971 (issued Oct. 2, 1984);

R is of type G) are disclosed in WO 86/00307 (published Jan. 16, 1986) and in U.S. Pat. 4,613,610 (issued Sept. 23, 1986); and

R is of type H) are disclosed in U.S. Applications Serial Nos. 919,275 (filed October 15, 1986) and 945,428 (filed December 22, 1986) as well as PCT World Patent application no 598/86 filed October 21, 1986;

said above-mentioned references being incorporated herein by reference thereto.

Since the compounds I are interconvertable by methods known in the art, each Compound I may also serve as an intermediate for other compounds I. For example, the $I_1$ esters ($R_1 = R_2$) may be saponified to obtain their corresponding compounds $I_2$ (in which $R_1$ = M, particularly where M = Na or K) and such salt forms acidified to yield corresponding Compounds $I_3$ (in which $R_1$ = H); and said Compounds $I_3$ may be cyclized to form their corresponding lactones of the formula $I_4$:

$I_4$.

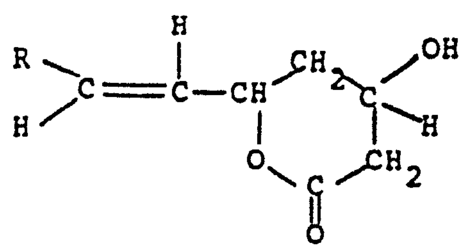

in which R is as defined above.

By the term "physiologically acceptable and hydrolyzable ester group" is meant a group which, together with the -COO- radical to which it is attached, forms an ester group which is physiologically acceptable and hydrolyzable under physiological conditions to yield a compound of Formula I wherein $R_1$ is hydrogen and an alcohol which itself is physiologically acceptable, i.e., non-toxic at the desired dosage level; particularly those which are free of centers of asymmetry. A preferred class of radicals as $R_2$ is an alkyl having from 1 to 4 carbon atoms, or benzyl, referred to herein as $R_2'$; and an especially preferred class being $C_{1-3}$alkyl, n-butyl, i-butyl, t-butyl and benzyl, collectively referred to as $R_2''$, eg ethyl.

M is preferably an alkali metal, such as sodium or potassium, or ammonium.

A compound $I_2$ in which $R_2$ is $R_2'$ can be converted to other esters within the definition of $R_2$, by well known means in the art, eg by transesterification.

This invention provides a novel method of preparing compounds I in optical isomer content approaching 100% purity, as well as improved methods of preparing intermediates for the preparation of such Compounds I.

Compounds I may be obtained by methods known in the art, employing as intermediates aldehydic compounds of the formula C:

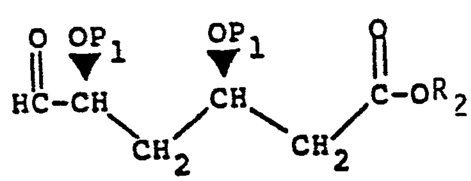

C

in which $P_1$ is a protective group and $R_2$ is as defined above, preferably $R_2'$ eg, a non-asymmeteric $(C_{1-4})$ alkyl such as ethyl. Such a procedure is represented in Reaction Scheme A, below, wherein $P_1$, R and $R_2$ are as defined above.

REACTION SCHEME A

C

Y

$I_1$

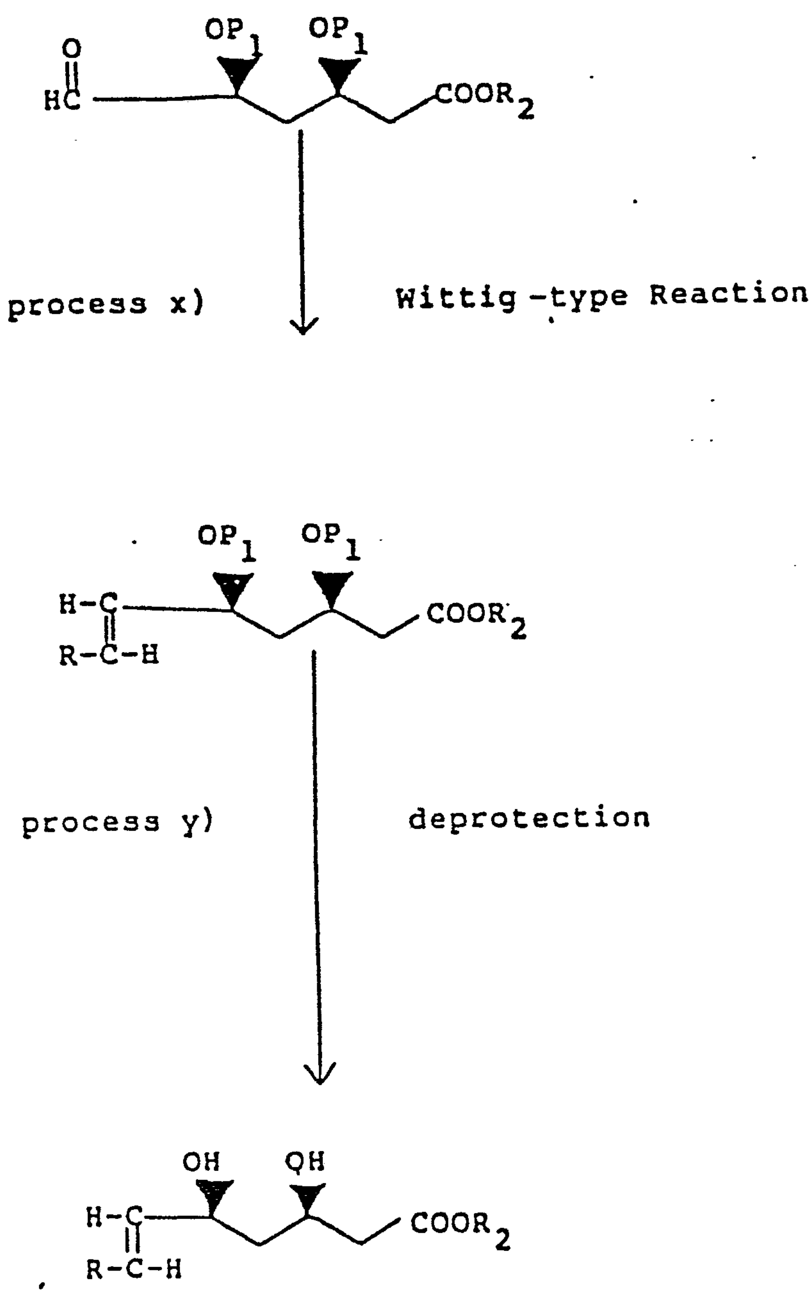

Preferred reagents for process x are those of Formula Q

$$R\text{-}CH{=}P(Rw)_3$$

or of Formula Qy

$$R\text{-}CH_2\text{-}\overset{\displaystyle O}{\overset{\|}{P}}\text{-}(ORy)_2$$

or of Formula W

$$R\text{-}CH_2\text{-}\underset{\displaystyle O}{\underset{\downarrow}{P}}\text{-}(OR_7)_2$$

wherein

R is as defined as above Rw is aryl expecially phenyl which is unsubstituted or substituted by one or two lower alkyl ($C_{1-4}$) or chloro substitutents, n-($C_{1-4}$) alkyl or cyclohexyl; Ry is n-($C_{1-4}$) alkyl expecially ethyl; $R_7$ is n-($C_{1-4}$)-alkyl or phenyl expecially methyl or ethyl.

Compounds C and their preparation are disclosed i.a. in USP 4,571,428 (issued February 18, 1986) as Compounds G.

The above-mentioned processes x) and y) of Reaction Scheme A, may be accomplished by methods described in the art, eg in above-mentioned USP 4,571,428, which patent is incorporated herein by reference. Processes x) and y) hereof are identified as processes g) and $h^1$) respectively, in said patent. Preparation of Wittig reagents (Q) is also described in said patent. Wittig reagents W and Qy can be prepared as hereinafter described or e.g. as described in WO 86/03488.

As mentioned above the compounds of Formula $I_1$ thus obtained can be converted to free forms or salt or lactone forms or to other esters eg as described in the references hereinbefore referred to.

An embodiment of this invention is a particularly advantageous method of preparing monoisomeric 3R, 5S-Compounds C by a novel multi-step procedure, starting from L-malic acid, which procedure may be conveniently represented by Reaction Scheme B, below.

In reaction scheme B, t is trityl; i.e. triphenylmethyl, $P_1$ is as defined above and $R_3'$ and $R_2°$ are radicals forming esters inert under the reaction conditions. $R_3'$ is for example n-$C_{1-6}$-alkyl preferably methyl or ethyl, $R_2°$ is for example n-$C_{1-6}$-alkyl eg $R_2'$ as defined above or allyl or any other pharmaceutically acceptable or non-pharmaceutically acceptable residue, $R_2°$ is especially methyl, ethyl or allyl.

It will be understood that during the preparation of final products of formula I from compounds C residues as $R_2°$ which are pharmaceutically unacceptable will be replaced by H, M or $R_2$ in conventional manner.

REACTION SCHEME B

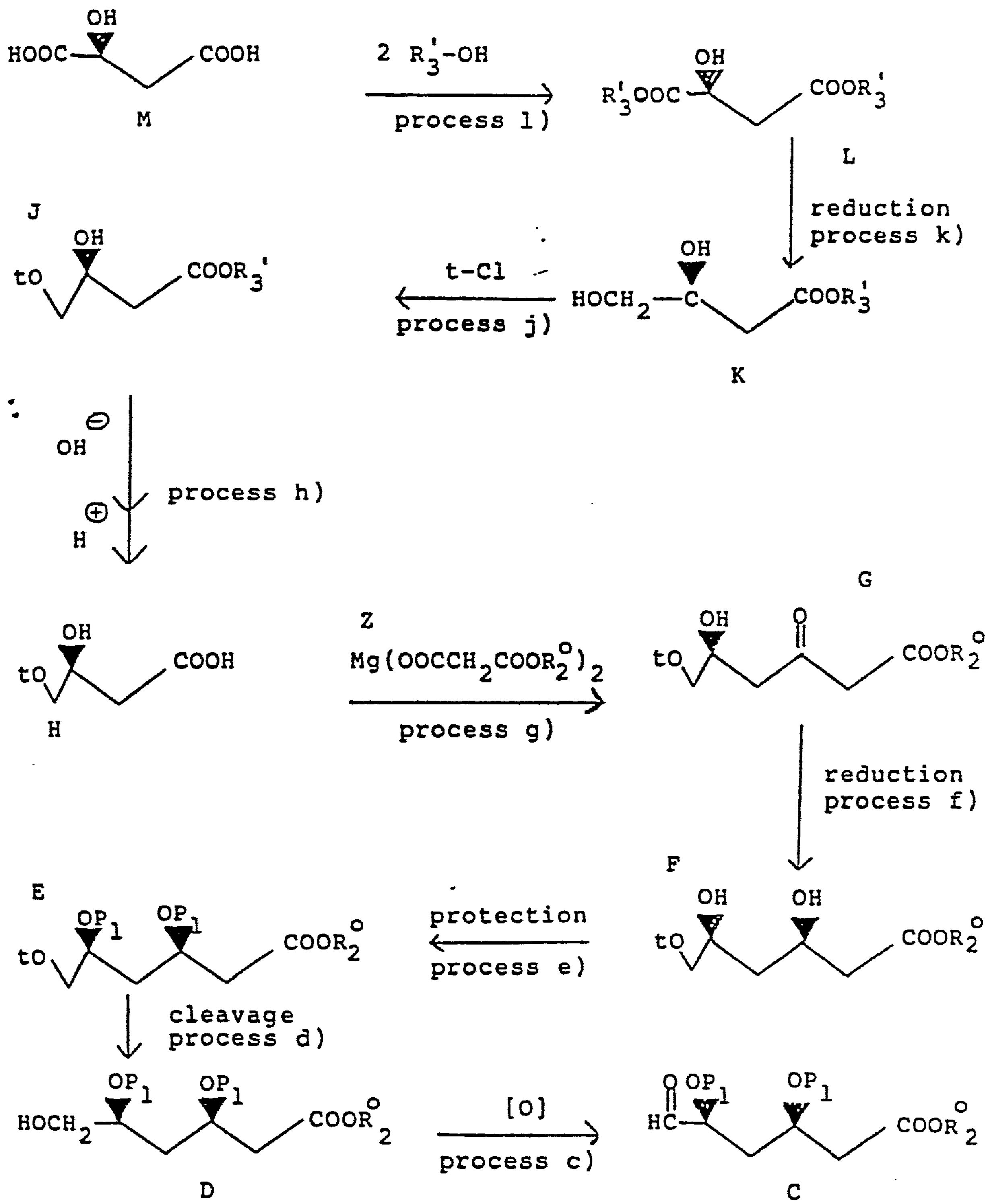

In the above-depicted procedure for preparing Compounds C, process l) is an esterification reaction, which may be accomplished by conventional methods of forming the di-esters of L-malic acid. It is particularly convenient to treat a charge of L-malic acid with at least two equivalents of alcohol, $R_3OH$, (preferably in large excess) at temperatures of from about -5° to 10°C, eg about 0 to 4°C, under acidic conditions eg provided by acetyl chloride, under essentially anhydrous conditions, the excess of the alcohol reactant serving reaction medium to yield the corresponding di-ester (L).

Process k) is a reduction, and may be accomplished, eg by treating the di-ester (L) with borane dimethylsulfide complex and a catalytic amount of sodium borohydride ($NaBH_4$), at from about 10° to 40°C, eg at room temperature (R.T.), under essentially anhydrous conditions in an inert medium, eg an ether, such as THF (tetrahydrofuran): dry alcohol ($R_3OH$) being added before recovery to "quench" the reducing agent, while avoiding contact with water during the recovery of the resultant hydroxy-monoester (K).

Process j) is an etherification of the free primary hydroxy function of a Compound K to obtain the corresponding trityloxy compound (J), such procedures being well known in the art. This may be accomplished by reacting a compound K with trityl chloride (eg in slight excess) in an inert solvent, eg a halogenated lower alkane, such as methylene chloride, in the presence of an acid acceptor, eg pyridine, at temperatures of from about 15° to 50°C, eg room temperature (R.T.).

Process h) consists of two phases, first a Compound J is saponified and then the resultant salt is neutralized with acid to obtain the corresponding Compound H. Such reactions may be carried out by conventional means; it being convenient to treat a Compound J with an aqueous alkali metal hydroxide, eg NaOH, in a co-solvent, eg THF, at moderate temperatures eg $10^{\circ}-80^{\circ}C$, such as R.T. The resultant salt may then be neutralized with a mild acid, eg citric acid, in situ.

Process g) is an homologation reaction in which Compound H is reacted with a magnesium salt of a malic acid monoester (reagent Z) to form the corresponding keto-ester (G). Process g) is carried out under essentially anhydrous conditions, at moderate temperatures eg R.T., in the presence of 1,1-carbonyldiimidizole in an inert medium, eg THF.

Reagents Z are obtainable by reacting two equivalents of a mono-ester of the formula Z'

Z' $$HO-\overset{O}{\overset{\|}{C}}-CH_2-\overset{O}{\overset{\|}{C}}-OR_2^{\circ}$$

in which $R_2^{\circ}$ is as defined above eg is as defined for $R_2'$ with an equivalent of magnesium methoxide (Process ma). The reaction may be carried out at moderate temperatures, eg R.T., in an inert polar medium, eg THF, under essentially anhydrous conditions. Compounds Z and Z' are known.

Process f) employs a three step process which involves first treating a compound Ga, with a trialkyl borane (Gr) (process $f_1$), then treating the reaction mixture with sodium borohydride (process $f_2$) to obtain a complex which is then cleaved (process $f_3$), to obtain the corresponding compound F.

The trialkyl borane reagent used in process $f_1$ has the formula Gr:

Gr $(R_4)_3B$

in which $R_4$ is a primary or secondary alkyl having from 2 to 4 carbon atoms.

Process $f_1$) is carried out under essentially anhydrous conditions, at about -80° to 30°C, eg at -70° or + 20 to 30°C, and the reaction medium consists essentially of a mixture of an alcohol of the formula $R_5OH$, in which $R_5$ is allyl or alkyl having from 1 to 4 carbon atoms, eg methyl or ethyl, preferably not tertiary/tetrahydrofuran (THF) in a ratio (v/v) of from about 1:3 to 6 of alcohol to THF, especially about 1:3 to 4.

Proportions of a Gr to compound G may be about 1-2.5 moles, preferably 1.02 to 1.3 moles:1.

Alternatively, in place of a trialkyl borane (Gr) there may be used an equivalent amount of a monoalkoxy dialkyl borane of the formula Gk:

Gk $R_6O-B-(R_4)_2$

in which $R_4$ is as defined above and $R_6$ is allyl or a lower alkyl having from 1 to 4 carbon atoms, preferably not tertiary. $R_4$, $R_5$ and $R_6$ may be the same, but need not be. Preparation of compounds Gk are described by Koster et al, Ann., 1975, 352 and Chen et al, Tetrahedron Letters 28, 155 (1987).

In the second stage, ($f_2$) the product of the first stage is treated *in situ* with sodium borohydride, initially at temperatures of about -70°C, and then allowed to warm to room temperatures (20° to 30°C). In the third stage ($f_3$) the product of the second stage, ie an intermediate of formula Gp and/or Gq:

Gp

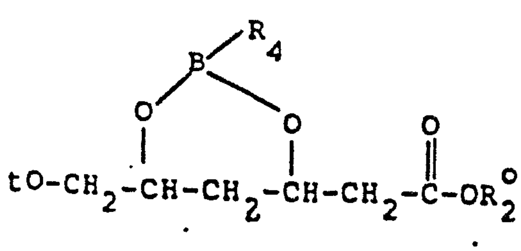

Gq

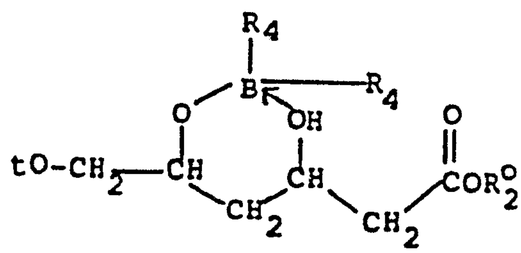

in which $R_2^o$, t, and $R_4$ are as defined, above, is dissolved in a polar organic solvent, eg a lower alkanol or THF or a mixture thereof, and treated with an aqueous hydrogen peroxide (e.g. 30%) solution at neutral pH, initially at a reduced temperature, eg about -70°C, and then the reaction mixture allowed to warm to a moderate temperature eg R.T., to obtain the corresponding compound F. Preferred alkanols are ethanol and methanol.

Alternatively, the product of process $f_2$ ) may be azeotroped with methanol or ethanol, preferably methanol, at, eg, from about 60° to 80° C, under essentially anhydrous conditions, to obtain the corresponding compound F.

It is particularly convenient to carry out all three stages of process f) at -70° C and in the same medium, using products of each stage *in situ*.

In forming reagents Gk *in situ* a preferred molar ratio of compound Gr per liter of alcohol is from about 0.2 to 1.2 mmol/ml., especially about 0.5 mmol/ml.

In Process e) the free hydroxy functions of a Compound F are protected, by known means.

Suitable protective groups $P_1$, include tri-substituted silyl radicals and have at least 2, and preferably 3 bulky radicals (eg 2 aryl and 1 alkyl), ie, radicals selected from the group consisting of a) tertiary alkyl ($C_4$ to $C_8$) groups especially t-butyl, and b) aryl, preferably phenyl which may be unsubstituted or substituted by up to 2 (preferabyl 0 or 1) of any of lower alkyl ($C_1$-$C_4$), chloro, nitro, trifluoromethyl, or mono-substituted in the para-position by a phenyl or benzyl (which may be unsubstituted or in turn substituted by one or two of such groups as recited above, especially at the para-position; non-bulky radicals being lower alkyl ($C_1$-$C_4$), eg methyl.

A preferred protective group is diphenyl-tert.-butylsilyl. While the protective groups need not be the same, it is more convenient that they be the same so that they can be introduced (and subsequently removed) at the same time.

Process e) may be accomplished by treating the diol (F) with at least two equivalents of a reagent of formula EP:

EP

$$P_1-L$$

in which $P_1$ is as defined above, and L is a leaving group such as chloro, bromo or p-methylbenzenesulfonyl, preferably chloro, eg diphenyl-tert.-butylsilylchloride, in the presence of an acid acceptor, eg imidazole, at eg 20 to 100°C, especially at 60°C, under essentially anhydrous conditions, in an inert medium, eg DMF.

Process d) is a selective cleavage, ie the trityl group is removed, while leaving the $P_1$ groups in place. This may be accomplished by known means eg by employing an acidic reagent. For example, $CF_3COOH$ at -78°C isd added to the di-protected ester (E) in an inert medium eg a halogenated lower alkane, such as methylene chloride/ water and then the reaction mixture held at about 0°C.

In Process c) the $-CH_2-OH$ unit of a Compound D is oxidized to an aldehydic function. Such reaction may be accomplished by known means. For example, a Compound D may be treated with pyridinium chlorochromate in the presence of molecular seives at moderate temperatures, eg R.T., in an inert medium, eg a halogenated alkane, eg methylene chloride.

Alternatively, compounds G in which $R_2'$ is t-butyl, may be obtained in one step from compounds J by treatment thereof with lithium t-butyl acetate (reagent Bp) under essentially anhydrous conditions at temperatures of from about -70° to -60°C. in an inert medium, eg THF. Reagent Bp may be prepared by known means, for example by treatment of t.-butyl acetate with lithium diisopropylamide reagent at, eg -65° to -70° C in an inert medium, eg THF. It is particularly convenient to carry out the preparation of reagent Bp and use it in situ in the same medium for reaction with compounds J, all under anhydrous conditions.

Preparation of Compounds Y via Process x) (Wittig Reaction) referred to in Reaction Scheme A, above, may be accomplished by known methods, care being exercized in the selection of materials and techniques to obtain a product especially rich in the trans-isomer, and then further enriching the product by known techniques, such as chromatography to obtain a Compound Y with as high a proportion of the desired isomer as possible for use in the following procedures. However, another embodiment of this invention is the improved synthesis of olefinic esters of the formula Y, as defined above, which are particularly rich in the trans isomer form, by the reaction of a compound of Formula C, defined above, with a phosphonate ester of the formula W

$$R-CH_2-\underset{\downarrow\atop O}{P}-(OR_7)_2 \qquad W$$

in which R is as defined above and $R_7$ is as defined above, ie Process x').

Reagents W are obtained by a two step reaction. First by halogenating an R-bearing of the formula W':

W' $$R-CH_2-OH$$

in which R is as defined above, so as to form the corresponding halide W''

W'' $$R-CH_2-Z$$

in which R is as defined above and Z is Cl or Br, depending on the halogenating agent used, preferably it is Cl. The halogenation may be carried out in the

conventional manner, eg using thionyl chloride where $Z = Cl$ is desired, at reduced temperatures, such as about $10^\circ$ to $60^\circ C$ eg mixing the reactants at about $0^\circ$, and eg then proceeding at R.T., in an inert medium, eg a corresponding halogenated lower alkane, eg methylene chloride when $Z = Cl$.

The second step involves reacting the resultant Compound W'' with a tri (lower alkyl) phosphite, eg trimethylphosphite, in an inert solvent, eg an aromatic hydrocarbon, such as toluene, with heating to $80^\circ$ to $140^\circ C$, eg at the reflux temperature of the reaction mixture.

Process x') employing as the Wittig reagent a Compound W, may conveniently be carried out in two stages. In the first stage a Compound W is reacted with a strong lithium base, eg a lithium salt of an organic compound, such as a lower alkyl lithium, especially n-butyl lithium, at reduced temperatures eg from about $-20^\circ$ to $0^\circ C$, in an inert medium, eg a cyclic ether, such as THF, under essentially anhydrous conditions, to form the corresponding lithium anion of the formula WA:

WA

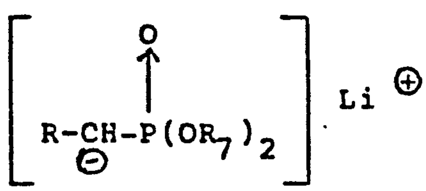

in which R and $R_7$ are as defined above. It is advantageous to include some lithium chloride.

In the second stage a reagent WA is reacted <u>in situ</u> with a Compound C at lower temperatures, eg from about $-20^\circ$ to $0^\circ C$, similarly in an inert medium eg THF, and under essentially anhydrous conditions.

The reaction mixture is then quenched, eg with acetic acid, and the resulting Compound Y isolated.

Deprotection of Compounds Y to their corresponding Compounds I, may be carried out in the conventional manner, eg by treating a Compound Y with at least 2 equivalents (eg 6) of acetic acid and tetrabutylammonium fluoride (TBAF) in THF, methanolic HCl or other fluoride anion reagents, at moderate temperatures, such as 20° to 60°.

An additional embodiment of this invention is a procedure for preparing Compounds $I_3$ ($R_2$=H) involving allyl ester intermediates. The procedure is conveniently represented in Reaction Scheme C, below in which R and $P_1$ are as defined above. Compounds $I_2'$ are sodium salts obtained where sodium hydroxide is employed in the second part of a standard deprotection process.

REACTION SCHEME C

C'

Y'

AA

$I_2$'

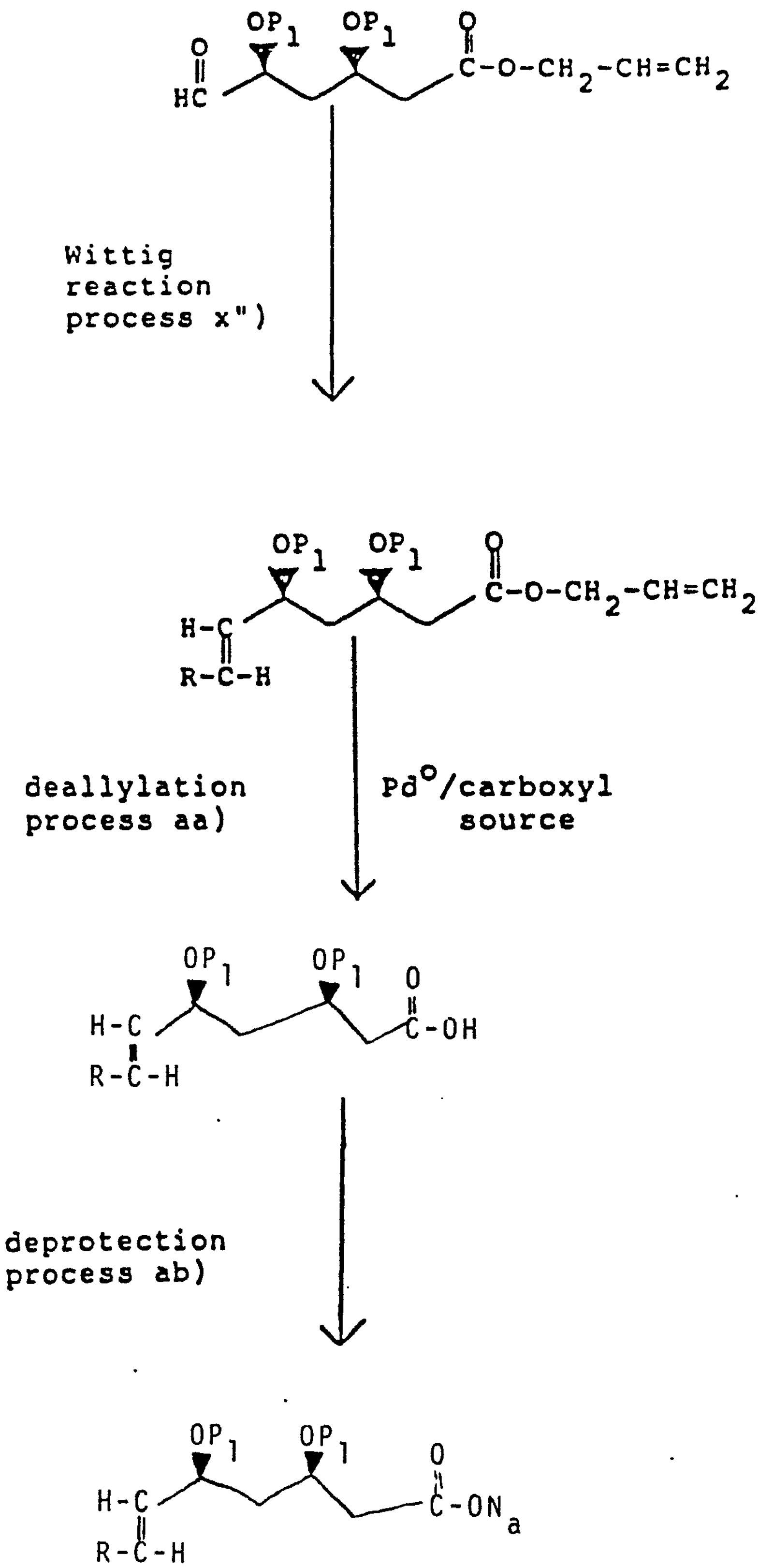

With reference to Reaction Scheme C, above, process x") is carried out analogously to process x) or x') described above. Compounds C' are compounds C in which the ester portion is allyl. Such compounds are obtainable in an analogous manner to the preparation of Compound C described above (via Reaction Scheme B) utilizing an allyl ester analog of a Compound Z) or by the method described in USP 4,571,428 using allyl alcohol in process e) thereof.

In process aa) the diprotected allyl esters of compounds $I_2$ are "deallylized", ie the allyl esters (Y') are converted to their parent carboxylic acid compounds (AA). The process aa) is carried out in the presence of soluble neutral palladium, eg, tetrakis triphenyl phosphine palladium complex, in the presence of carboxylic material, eg acetic acid or ammonium formate, in a medium eg methylene chloride or a cyclic ether, such as dioxane or THF at moderate temperatures, eg 20° to 90°; conveniently at room temperature or the reflux temperature of the mixture. The preferred palladium source is tetrakis (triphenyl phosphinyl) palladium:

Pd 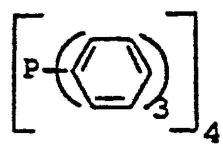

Deprotection (process ab) may be carried out in the same manner as in process y) described above. Once a compound $I_2$ is obtained it can be converted to other forms of compounds I, as described above.

A distinct advantage of the procedure of Reaction Scheme C is that compounds $I_2$ are obtained with little or no concurrent lactone ($I_4$) formation, the presence of which complicates refining operations. In addition, deprotection proceeds smoothly and gives high yields of the erythro isomer.

Reagents and starting materials described herein, e.g., compounds Q, Z' , EP, W' , W" , Gr, Gk and Bp are known and obtainable by known means, or where not known, may be obtained by adaptation of methods reported in the literature for the preparation of known analogs; some compounds being commercially available.

The final products and intermediate compounds described herein may be recovered and refined, where such is desired, by conventional means, such as by crystallization, distillation or chromatographic techniques such as column or thin layer chromatography, e.g., silica gel column chromatography.

The following examples are illustrative of the invention. All temperatures are centigrade and room temperature (R.T.) is 20 to 30°C, unless indicated otherwise. Evaporations are done under vacuum employing minimal heating. Drying of organic phases is done over anhydrous magnesium sulfate, unless indicated otherwise. THF is tetrahydrofuran, DMF is dimethyl formamide, TFA is trifluoroacetic acid, and TP is tetrakis (triphenyl phosphinyl) palladium.

Preparation 1

Magnesium bis-(mono-allyl malonate)

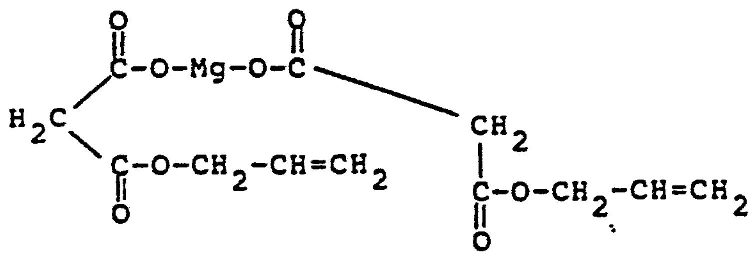

Step 1, mono-allyl, mono-acid malonate

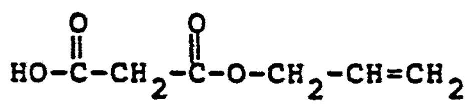

8 ml of 2N aqueous potassium hydroxide was added to a solution of 2.908g (15.8 mmol) of diallyl malonate in 8 ml of allyl alcohol at 0°. The reaction mixture was stirred at room temperature for two hours at which time the pH value of the solution was 7 to 8. The solution was concentrated under reduced pressure to obtain a residue, and the residue was diluted with ether and water. The organic layer was discarded, and the aqueous layer was acidified by 2N hydrochloric acid until it became pH 2-3 and then saturated with solid sodium chloride.

The aqueous layer was then extracted with ethyl acetate. The combined organic layers were washed once with 20 ml of saturated aqueous sodium chloride solution, dried over anhyd. sodium sulfate and filtered. Removal of the organic solvent under reduced pressure afforded 2.043g of the title mono-ester of this step.

Step 2, magnesium bis-(mono-allyl malonate)

6.693g (46.5 mmol) of the mono-ester of Step 1, above, was dissolved in 116 ml of dry THF and 2g (23.2 mmol) of magnesium methoxide was added to this solution at room temperature. The mixture was stirred for one hour, and it was placed on rotavapor to remove solvent under reduced pressure to give 7.163g of the title product.

Repeating the procedure of this preparation but starting with an approximately equivalent amount of

a) diethyl malonate; or

b) dimethyl malonate

in place of the diallyl malonate and in place of the allyl alcohol,

a) ethanol; or

b) methanol,

used herein, there is analogously obtained:

a) magnesium bis-(monoethyl malonate); and

b) magnesium bis-(monomethyl malonate), respectively, which are used in Example 1, below.

Preparation 2

[[1-(4-fluorophenyl)-4-isopropyl-2-phenyl-1H-imidazol-5-yl]methyl]-phosphonic acid, dimethyl ester

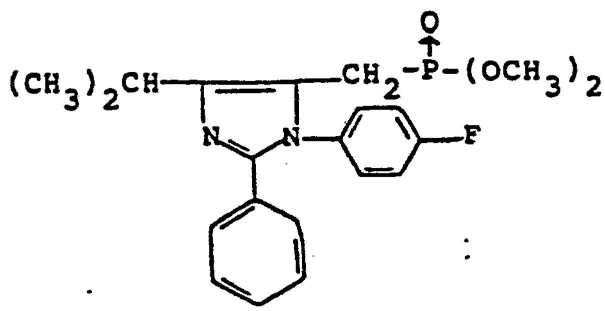

A solution consisting of 1.25 liters of methylene chloride and 310.5 grams of 1-(4-fluorophenyl)-4-isopropyl-2-phenyl-1H-imidazole-5-methanol is cooled to 0°C. 178.4 grams of thionyl chloride is added to the reaction mixture over a period of several minutes, while maintaining an internal temperature of 10°C. The reaction contents are stirred at room temperature for about 16 hrs. Under a reduced pressure of 30-50 mm and a maximum internal temperature of 40-45°C as much of the solvent as possible is removed by distillation. There is then added to the reaction mixture 250 ml of toluene and the distillation continued. At atmospheric pressure there is then added to the reaction mixture 200 ml of toluene. The reaction contents are heated to 90-95°C. Over a period of 30-60 minutes, there is slowly added 1.32 kg of trimethylphosphite. The reaction mixture is heated and held at reflux for two hours. The distillable solvents are removed by distillation at 20-40 mm to obtain a residue. To the resulting solid residue is added 250 ml of toluene and the distillation continued until no further distillate can be recovered. The resulting solid is crystallized from 1.1 liters of toluene to obtain 233 grams of the title product, mp 139-142°.

Preparation 3

[[1-(3,5-dimethylphenyl)-3-methyl-2-naphthalenyl]methyl]-phosphonic acid dimethyl ester

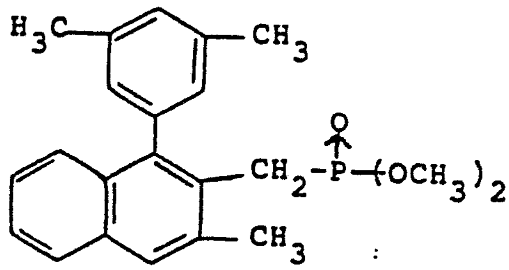

To 21 ml of methylene chloride is added 16.7 grams of 1-(3,5-dimethylphenyl)-3-methyl-2-naphthalene methanol. The reaction mixture is cooled to 17° and 4.6 grams of thionyl chloride is added slowly while maintaining a maximum internal temperature of 24°. The reaction mixture is stirred for 2 hours. By distillation, under a reduced pressure of 20-30 mm and an outside temperature of 65° solvents are removed and there is recovered a thick oil as residue. To the residue is added 50 ml of toluene and the distillation continued until all the solvent is recovered. The addition of toluene and the distillation is repeated two more times, yielding a thick oil. To the thick oil, is added 35 ml of trimethylphosphite. The reaction mixture is heated to reflux, (115-120°) for 20 hours. The reaction mixture is then concentrated under reduced pressure until no trimethylphosphite can be recovered by distillation. To the reaction contents is added 30 ml of heptane and the distillation continued under reduced pressure. The addition of heptane is repeated several times. All solvents are distilled off to leave crude title product as an orange solid residue. The residue is crystallized from heptane to yield 16 grams of the title product, mp 132-135°.

## Preparation 4

[[1'-isopropyl-3'-(4"-fluorophenyl)-indol-2'-yl]methyl] phosphonic dimethyl ester

To 2.5 ml of dimethylformamide in 500 ml of dry THF, is added 50 grams of 3-(4-fluorophenyl)-1-isopropyl-1H-2-indolemethanol. To the reaction mixture is added, (over a period of 20 minutes) 50 ml of oxalyl chloride while maintaining a maximum temperature of 25°. The reaction contents are stirred at R.T. for two hours.

Under a reduced pressure of 20-30 mm and an external temperature of 40°C as much solvent as possible is recovered by distillation. 250 ml of toluene is added to the remaining reaction mixture and the distillation is continued until no solvent distills off. To the reaction contents , 100 ml of trimethyl phosphite are added, and the reaction mixture heated to reflux for 1.5 hours. Under a reduced pressure of 20-30 ml, all the solvents are removed and 250 ml of toluene is then added and distillation continued until no solvent distills off leaving an oily residue. 39 g of the title product is crystallized from the oil (from hexane) mp 94-6°.

Example 1

(3R-5S)-bis-(diphenyl-tert.-butylsiloxy)-6-oxo-hexanoic acid, ethyl ester

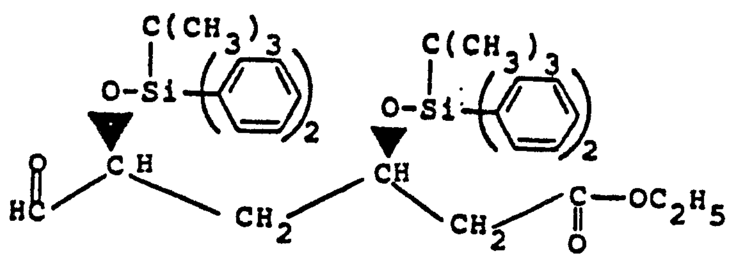

Step 1, dimethyl L(-)malate

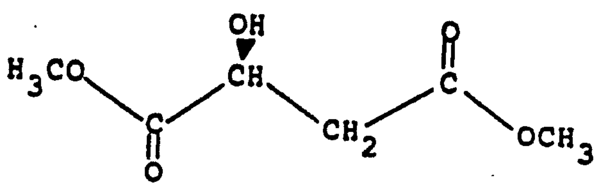

Into a vessel charged with 1356 ml of methanol was added dropwise 67.8 ml (78.5 g/mol) acetyl chloride at 3-4°C. After complete addition, the solution was stirred for one hour at 0°. 176g (134 g/mol) L-malic acid was introduced to the solution in portions over a period of 35 minutes. The reaction mixture was stirred at 0° for 2 hours, then for about 18 hours at room temperature, and then concentrated in vacuo. Distillation of the residue was performed under vacuum to give the title product of this step (173.82g (b.p. 78°C - 86°C/0.77 mm).

Step 2, methyl 3S,4-dihydroxybutyrate

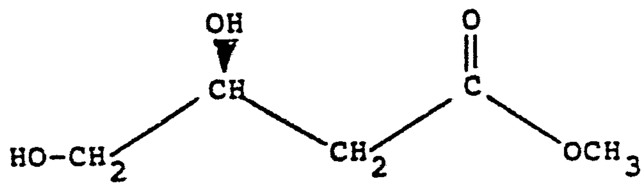

To a vessel, charged with 19.2g (0.12 mole) hydroxy-diester of step 1, above, in dry THF (250 ml) was added dropwise 12.2 ml of a 10M solution of borane-dimethyl sulfide (0.122 mol), under a nitrogen atmosphere, and the mixture was stirred at 16-20° for 0.5 hours, (which evolved hydrogen gas). Then 0.2g $NaBH_4$, (6 mmol) was added to the mixture and the resulting solution was stirred for an additional 0.5 hour. During this period the temperature was increased from 20° to 35°, followed by the addition of 77 ml of dry methanol; stirring being continued for 0.5 hour. The solvent was removed by aspirator first and then evaporated under reduced pressure to give 17.34g of crude product of this step, which was purified by column chromatography (5 x 18.5 cm) using ethyl acetate:hexane; 2:1 as initial elutant and ethyl acetate as final elutant, to obtain 14.556g of refined title product of this step.

Step 3, methyl 4-trityloxy-3S-hydroxy butyrate

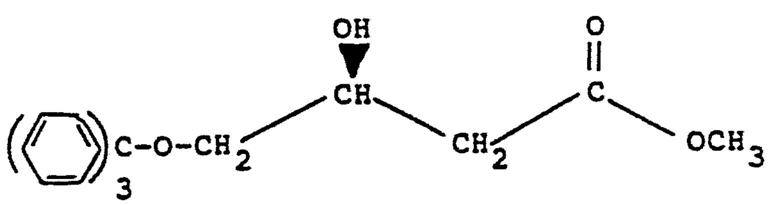

To a 25.75g (0.192 mol) of the product of step 2, above, dissolved in 257 ml of pyridine was added dropwise 56.25g (0.201 mol) trityl chloride dissolved in 172 ml of methylene chloride at 0°. After complete addition, the reaction mixture was warmed up to room temperature and stirring continued for about 16 hours. Removal of solvent under reduced pressure gave a residue which was dissolved in ethyl acetate. The organic layer was washed with 2N hydrochloric acid to remove pyridine, followed by washing with saturated aqueous solution of sodium bicarbonate, dried over anhyd. magnesium sulfate and filtered. The solvent was evaporated to give the crude title product of this step, which was refined by column chromatography using ethyl acetate:hexane; 1:18 as elutant to obtain 61.4g of the title product of this step as solid [α] = -4°.

Step 4, 3S-hydroxy-4-trityloxy-butyric acid

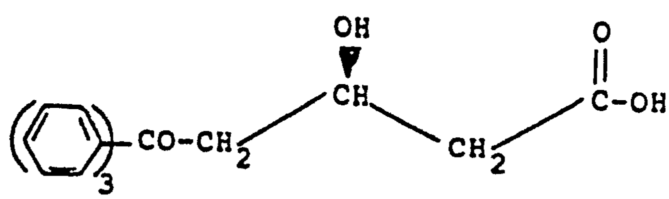

7.227g (19.22 mmol) of the hydroxyester product of Step 3 was dissolved in 20 ml of THF at 0° and 20 ml of 1N aqueous sodium hydroxide solution (20 mmol) was added dropwise to the solution at 0°. After completion of addition, the resulting solution was warmed and stirred at room temperature for two hours, (at which time TLC indicated no starting material). Citric acid (25 ml, 28% in water solution) was poured into the solution to neutralize. The aqueous layer was extracted 3 times with 150 ml portions of methylene chloride. The combined organic layers were dried over anhyd. magnesium sulfate to give product of this step, which was used directly for next step.

Step 5, ethyl 5S-hydroxy-6-trityloxy-3-oxo-hexanoate

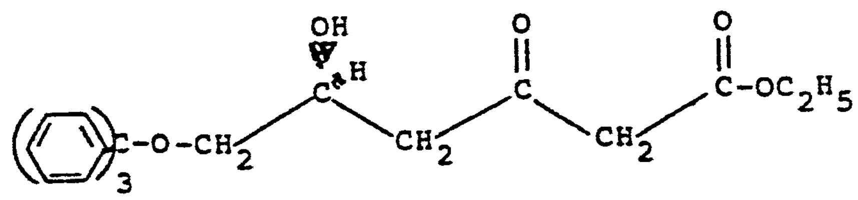

0.685g (4.228 mmol) of 1,1-carbonyldiimidazole was added to a solution of 1.392g (3.84 mmol) of the hydroxy-acid product of step 4, above, in 19.2 ml of anhydrous THF at 0°. The resulting solution was stirred at room temperature for four hours, then 1.218g (4.228 mmol) of Mg $(COO-CH_2-COOC_2H_5)_2$ was added to the solution at same temperature and the reaction mixture was stirred for about 16 hours. The solvent was then evaporated under reduced pressure to obtain a residue which was dissolved in ethyl acetate. 11 ml of a 25% citric acid solution was then added to the mixture to acidify it. The aqueous layer was extracted with another 90 ml of ethyl acetate. The combined organic layers were washed twice with 25 ml portions of aqueous sodium bicarbonate (10%) or until the aqueous layer became basic, the extract was dried over anhyd. magnesium sulfate and filtered. Solvent was removed under reduced pressure to give crude title product of this step which was refined by column chromatography using ethyl acetate:hexane; 1:4 as elutant to yield 0.725g of the title product of this step [α]D -10.14°.

Step 6, ethyl (Syn) 3,5-dihydroxy 6-trityloxy hexanoate

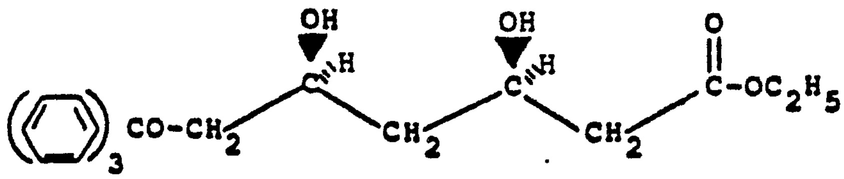

0.429g (0.988 mmol) of the hydroxy-keto-ester product of step 5, above, dissolved in 8 ml anhydrous THF and 2 ml ethanol was mixed with 2.2 ml of 1M triethylborane (in hexane) at room temperature. (No heat was generated). The resulting solution was stirred at room temperature for two hours, and then 41.61 mg of sodium borohydride was added at -78°. The reaction mixture was stirred at room temperature for 5 hours. (TLC showed there was a trace of starting material). The solution was poured into a solution of 17.24 ml ethanol, 17.24 ml of pH=7.00 buffer solution and 8.62 ml of 30% aqueous hydrogen peroxide at -70°C. After removal of the cooling, the solution was stirred for half an hour at R.T. Most of organic solvent was then evaporated under reduced pressure to obtain a residue. The residue was extracted twice with portions of methylene chloride, and the combined organic layers were dried over anhydrous magnesium sulfate, filtered, and evaporated to give the crude title product of this step which was refined by column chromatography to yield refined the title diol-ester of this step. (348 mg), [α]D = -4.72° (about 98% syn).

Repeating this step but using in place of the triethylborane, an approximately equivalent amount of monomethoxy, diethylborane, the same product is obtained (about 98% syn isomer).

Step 7, ethyl (Syn)-3,5-di-(diphenyl tert.-butylsiloxy)-6-trityloxy-hexanoate

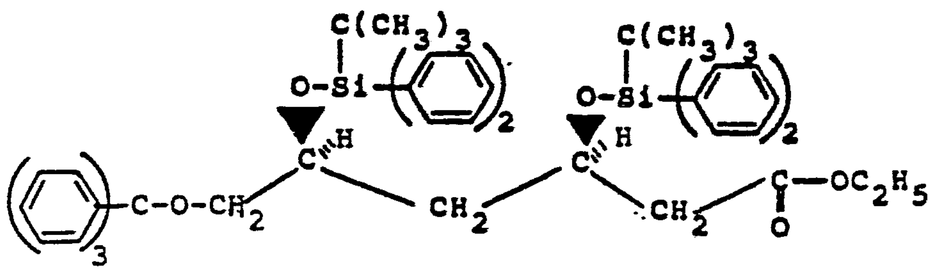

0.263g (0.6 mmol) of the diol- ester product of step 6, above, was dissolved in 2.5 ml of DMF and 0.205g (5 equivalents) of imidazole was added to this solution. The mixture was stirred for ten minutes, and then 0.373g (1.32 mmol) of t-butyl-diphenylsilyl chloride was introduced dropwise to the solution by syringe. After stirring for 4 hours, the reaction mixture was heated to 60° and continued to stir for 18 hours. The mixture was then diluted with ethyl acetate and washed three times with water. The organic layer was dried over as anhydrous magnesium sulfate, and filtered. Removal of solvent gave crude title product of this step, which was refined by column chromatography using ethyl acetate=hexane; 1:25 as elutant to give 480 mg of refined title product of this step [α]D = -24.553°.

Step 8, ethyl(Syn)-3,5-di(diphenyl tert.-butylsilyloxy)-6-hydroxy-hexanoate

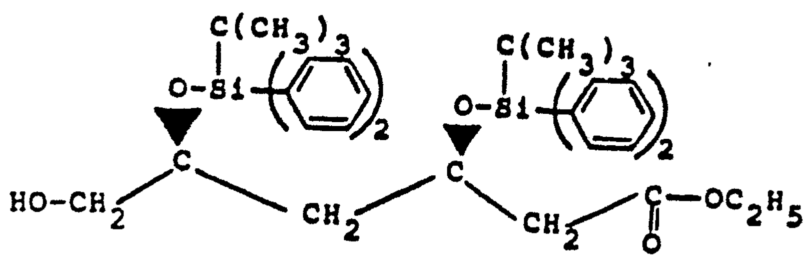

0.443g (0.486 mmol) of the disiloxy-ester product of step 7, above, is dissolved in 5 ml of methylene chloride and mixed with 0.5 ml of 70% aqueous trifluoroacetic acid (v/v) at -78°. After complete addition, the reaction mixture was warmed up to 0° and stirred at that temperature for three hours. The reaction mixture was then diluted with ethyl acetate and washed with saturated aqueous sodium bicarbonate solution. Removal of the organic solvent gave crude title product of this step, which was diluted with hexane to precipitate out triphenyl methanol without contaminating with any of the desired product. The solid was filtered off and the mother liquid was concentrated _in vacuo_ to yield a crude mixture which was refined by column chromatography using ethyl acetate:hexane = 1:10 as elutant to provide 0.272g, of the title product of this step, [α]D - 7.8°.

Step 9, 3R,5S-bis-(diphenyl-tert.-butylsiloxy)-6-hexanoic acid, ethyl ester

0.239g (0.357 mmol) of the hydroxy product of step 8, above, was dissolved in 2.5 ml of methylene chloride. 0.48g of molecular sieve (4A°) was added to the solution and then 0.24g (1.11 mmol) of pyridinium chlorochromate was added to the reaction mixture at room temperature. The resulting mixture was stirred at room temperature for one hour. The whole mixture was placed on the top of a filter funnel containing celite and the product was eluted out by methylene chloride to give 215 mg of the title product of this example, [α]D = +1.31°.

Repeating the procedure of this example but in Step 5 using in place of the magnesium bis-(monoethyl malonate) an approximately equivalent amount of:

a) magnesium bis-(monoallyl malonate) or
b) magnesium bis-(monomethyl malonate) (obtained by Preparation 1 above),

and employing in place of the ethanol in Step 6, an equivalent amount of:

a) allyl alcohol or
b) methanol, respectively,

there is accordingly obtained:

a) 3R, 5S-bis-(diphenyl-tert.-butylsiloxy)-6-oxo-hexanoic acid, allyl ester; and
b) 3R, 5S-bis-(diphenyl-tert.-butylsiloxy)-6-oxo-hexanoic acid, methyl ester, respectively.

Example 2

(E)-Sodium erythro-7-[1'-isopropyl-3'-(4"-fluorophenyl) indol-2'yl]-3,5-dihydroxyhept-6-enoate

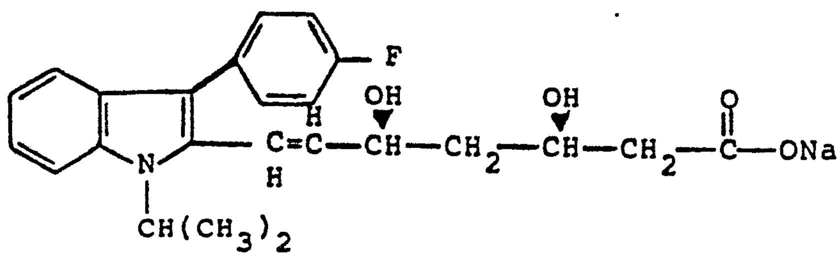

Step 1, allyl, erythro-3,5-di-(diphenyl-t-butylsiloxy)-6-hydroxy-hexanoate (racemic)

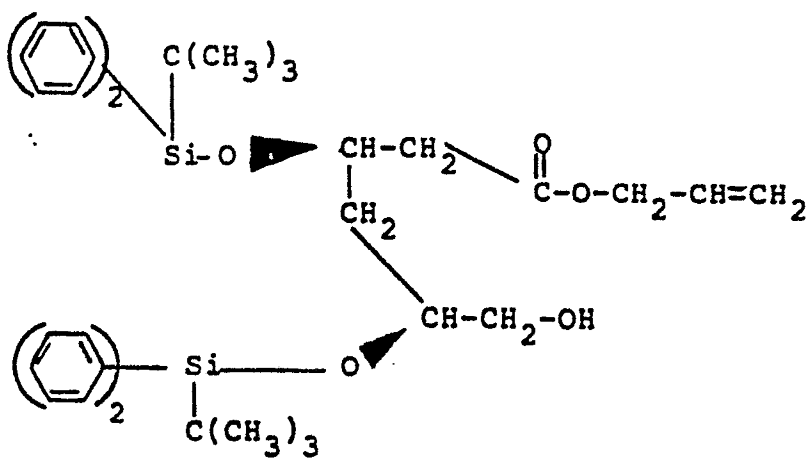

To 132 ml of allyl alcohol is added 1 ml of TFA and 30g of cis-2-oxy-4,6-di-(diphenyl-t.-butylsiloxy)-cycloheptanone. (Disclosed in U.S.P. 4,571 ,428 at Step D of Example 1). The mixture is heated to reflux (internal temp. about 97°) for from 5 to 6 hours. The mixture is then cooled to room temperature. The small amount of solid present is removed by filtration and the filtrate evaporated to dryness to obtain as a residue the title hydroxy product

of this step. To the residue is added water and methylene chloride and the product extracted (separation is slow because of emulsion). The refined product is recovered from the extract, by evaporating off solvent, for use in the next step.

Step 2, erythro-3,5 di-(diphenyl-t.-butylsiloxy)-6-oxo-hexanoic acid, allyl ester (racemic)

To a vessel are added 1.36g (2 mmol) of the hydroxy-product of step 1, above, 1.08g (5 mmol) of pyridinium chlorochromate and 2.00g of type 4A molecular sieve powder, and 30 ml of methylene chloride, and the mixture stirred at R.T. for 2 hours. The mixture is then filtered on silica gel and the filtrate evaporated to obtain as a residue the title aldehyde product of this step, for use in the next step.

Step 3, (E)-allyl, erythro-7-[1'-isopropyl-3'-(4-fluorophenyl)indol-2'-yl]3,5-di-(diphenyl-t.-butylsiloxy)-hept-6-enoate

0.75g (2 mmol) of [1'-isopropyl-3'-(4'-fluorophenyl)indol-2'-yl]methyl-phosphonic acid dimethyl ester obtained by Preparation 4, above, is dissolved in 5 ml of dry THF, and the mixture cooled to -20°. 1.4 ml of 1.5 molar solution of n-butyl lithium (in THF) is then added and the mixture is stirred at -20° for 20 minutes.

To the mixture is then added (dropwise, 1.34g (2 mmol) of erythro-3,5-di-(diphenyl-t.-butylsiloxy)-6-oxo-hexanoic acid, allyl ester (of step 2, above) in 3 ml of dry THF, and the mixture is stirred for 5 hours at 0°.

The reaction mixture is diluted with 50 ml of methylene chloride, washed with saturated aqueous ammonium chloride solution, the organic phase is dried over anhyd. magnesium sulfate, and evaporated to obtain a residue containing crude title product of this step. The residue is chromatographed on silica gel eluting with hexane/ethyl acetate (4:1) to recover refined title product of this step (in about 50% yield).

Step 4, (E)-erythro-7-[1'-isopropyl-3'-(4"-fluorophenyl) indol-2'-yl]-3,5-(diphenyl-t-butylsiloxy)-hept-6-enoic acid

A mixture of 0.90g (1 mmol) of the allyl ester of step 3, above, 3 ml of glacial acetic acid, 4 mg of triphenyl phosphine, 40 mg of palladium tetra (triphenylphosphine) and 3 ml of methylene chloride are stirred at R.T. for 1 hr. The mixture is then evaporated to dryness to obtain a residue. Chromatographing the residue on silica gel using hexane:ethyl acetate (4:1) as eluant yields refined title product of this step (in about 56% yield).

Step 5, (E)-Sodium erythro-7-[1'-isopropyl-3'-(4"-fluorophenyl)indol-2'yl]-3,5-dihydroxyhept-6-enoate

20 ml of 1M tetrabutylammonium fluoride (in THF) is mixed with 1.2 ml of glacial acetic acid. To the resulting solution, 1.774g (2 mmol) of the diprotected acid of step 4 above, is added and the resulting mixture is stirred for about 50 hours at ambient temperatures.

The reaction mixture is then diluted with 200 ml of ethyl acetate, and washed four times with water. The organic phase is dried over anhyd. magnesium sulfate and evaporated to obtain a residue. The residue is partitioned between water and hexane, and a solid results. The solid is the free acid of the title product, and is recovered by filtration (in about 100% yield).

The free acid is converted to its sodium salt by conventional means.

Repeating the procedure of step 4 of this example using techniques described above in Table A, improved yields are obtained.

Repeating the procedure of this example, using as W in step 3, the phosphonate esters of a) Preparation 2, b) Preparation 3, above, or c): [1'-isopropyl-3'-(3",5"-dimethylphenyl)indol-2'-yl]methyl phosphonic dimethyl ester (prepared analogously), there is accordingly obtained a) (E)-<u>erythro</u>-7-[1'-(4"-fluorophenyl)-4'-isopropyl-2'-phenyl-1H-imidazol-5-yl]-3,5-dihydroxyhept-6-enoic acid, and the sodium salt thereof; b) (E)-<u>erythro</u>-7-[1-(3,5-dimethylphenyl)-3-methyl-2-naphthalenyl]-3,5-dihydroxyhept-6-enoic acid, and the sodium salt thereof; and c) (E)-<u>erythro</u>-7-[1'-isopropyl-3'(3", 5"-dimethylphenyl)indol-2'-yl]3,5 dihydroxyhept-6-enoic acid, and the sodium salt thereof.

Repeating the procedure of this example, but using in place of the racemic <u>erythro</u> 3,5-di(diphenyl-t.-butylsiloxy)-6-oxo-hexanoic acid, allyl ester the 3R,5S-chiral form obtainable by Example 1, there is accordingly obtained the title product of this example in 3R,5S-chiral form.

## Example 3

Repeating the procedure of Step 4 of Example 2, but using in place of the glacial acetic acid/triphenyl phosphine/palladium tetra (triphenyl phosphine) system used therein an equivalent amount of the systems of Table A below, as well as variations in reaction conditions noted therein, the results are obtained as reported therein.

### TABLE A

| Run No. | $Pd^{O}$ Source | Carboxylate | Medium | Temperature | Time (Hours) | % Yield |
|---|---|---|---|---|---|---|
| 1 | TP | $CH_3COOH$ | $CH_2Cl_2$ | RT. | 18 | About 50 |
| 2 | $PD(OCCH_3)_2$ + 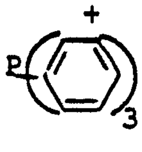 | $HCOONH_4$ | Dioxane | Reflux | 2 | 10 |
| 3 | TP | $HCOONH_4$ | Dioxane | Reflux | 1 | 85 |
| 4 | TP supported on polystyrene | $HCOONH_4$ | THF | Reflux | 18 | 85 |

Example 4

t.-butyl 5S-hydroxy-6-trityloxy-3-oxo-6-hexanoate

Under a nitrogen atmosphere at 0°, 87.78 ml of 1.5M (132 mmole) of n-butyllithium is added dropwise to 18.49 ml (132 mmole) of diisopropylamine in 150 ml of dry THF, and the mixture stirred for 30 mins. to form lithium diisopropylamide reagent.

The reagent solution is cooled to -70°, and a solution of 17.76 ml (132 mmole) of t-butyl acetate in 25 ml of dry THF added to the reagent solution over a period of one hour at -65° to -70° to form lithium t-butyl acetate. After complete addition, the cooling is maintained and the resulting solution is stirred for 1.5 hour. 11.28 g (30 mmole) of methyl 4-trityloxy-3(S)-hydroxy butyrate (obtainable by step 3 of Example 1) in 75 ml of dry THF is added to the solution of lithium t-butyl acetate at -65° to -70°. After complete addition of the trityl ester, the resulting yellow solution is stirred at about -70° for 0.5 hours, then it is warmed to -10° and stirred at -10° for one hour (with TLC monitoring to indicate the reaction is over). 150 ml of saturated aq. ammonium chloride is added to quench the reaction at -60° to -70°, and then the mixture warmed to 0°. The entire mixture is then transferred to a separatory funnel and diluted with ethyl acetate and the organic phase separated.

The aqueous layer is then extracted with an additional 200 ml of ethyl acetate and the organic phases combined, washed with 90 ml of 1N hydrochloric acid, 90 ml of saturated aq. sodium bicarbonate, dried and filtered. The organic extract solution is then evaporated to yield 17.43 g of crude product (estimated to be about 80% of the title product of this step, based on chromatographic analysis).

Example 5

(E) erythro-7-[1'-4"-fluorophenyl)-4'-isopropyl-2'-phenyl-1H-imidazol-5-yl]-3R,5S-dihydroxyhept-6-enoic acid, sodium salt

Repeating the procedure of Step 2 of Example 2, above, but using in place of the erythro-3,5 di-(diphenyl-t.-butylsiloxy)-6-oxo-hexanoic acid, allyl ester (racemate) an approximately equivalent amount of:

a) (3R-5S)-bis-(diphenyl-tert.-butylsiloxy)-6-oxo-hexanoic acid, allyl ester;
b) (3R-5S)-bis-(diphenyl-tert.-butylsiloxy)-6-oxo-hexanoic acid, ethyl ester; or
c) (3R-5S)-bis-(diphenyl-tert.-butylsiloxy)-6-oxo-hexanoic acid, methyl ester (obtainable according to Example 1), or
d) the t-butyl ester aldehyde title product of Example 4, above, and in place of the indol phosphonate ester of Preparation 4, an approximately equivalent of the imidazol phosphonate ester of Preparation 2, there is accordingly obtained respectively the allyl, ethyl, methyl and t.-butyl ester forms of erythro-7-[1'-4"-fluorophenyl)-4'-isopropyl-2'-phenyl-1H-imidazol-5-yl]-3R-5S-di-(diphenyl-t-butylsiloxy)-6-hept-enoic acid, which upon deprotection respectively yield the corresponding:

a) allyl;
b) ethyl;
c) methyl; and
d) t.-butyl ester form of the title acid salt, each of which upon saponification; or hydrolysis followed by sodium salt formation in the conventional manner, eg analogous to the manner described in USP 4,571,428 and WO 84/02131 accordingly yields the title product.

Title compound: m.p. 207-10(discolor.). $\alpha_D^{25}$=+18.31 (0.7 $CH_3OH$)
Methyl ester : m.p. 139-141. $\alpha_D^{25}$ +41.51 (2.28 $CH_3OH$)
t-Butyl ester : m.p. 144-5 $\alpha_D^{25}$ +8.48 (2.19 $CH_2Cl_2$)

Example 6

(E)erythro-7-[1'-isopropyl-3'-(4"-fluorophenyl)-indol-2'-yl]-3R, 5S-dihydroxyhept-6-enoic acid, sodium salt

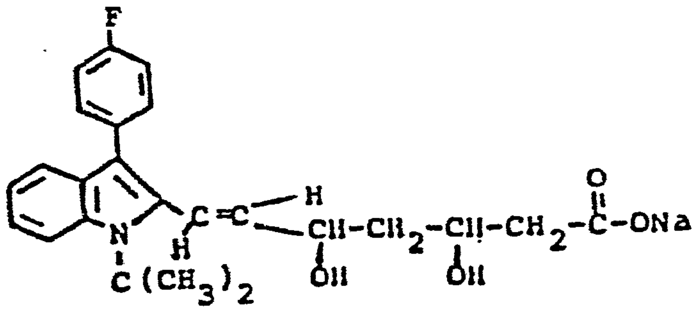

Repeating the procedure of Step 2 of Example 2, above, but using in place of the erythro-3,5 di-(diphenyl-t.-butylsiloxy)-6-oxo-heptenoic acid, allyl ester (racemate) an approximately equivalent amount of:

a) (3R-5S)-bis-(diphenyl-tert.-butylsiloxy)-6-oxo-hexanoic acid, allyl ester;
b) (3R-5S)-bis-(diphenyl-tert.-butylsiloxy)-6-oxo-hexanoic acid, ethyl ester; or
c) (3R-5S)-bis-(diphenyl-tert.-butylsiloxy-6-oxo-hexanoic acid, methyl ester (obtainable according to Example 1), or
d) the t-butyl ester aldehyde title product of Example 4, above, there is accordingly obtained respectively the allyl, ethyl, methyl and t.-butyl ester form of erythro-7-[1'-isopropyl-3'-(4"-fluorophenyl)indol-2'-yl]-3R-5S-di-(diphenyl-5,-butylsiloxy)-6-enoic acid, which upon deprotection respectively yields the corresponding:
a) allyl;
b) ethyl;
c) methyl; and
d) t.-butyl ester form of the title acid salt, each of which upon saponification; or hydrolysis followed by sodium salt formation in the conventional manner, eg analogous to the manner described in USP 4,571,428 and WO 84/02131 accordingly yields the title product.

Example 7

Repeating Step 6 of Example 1, but carrying out the first stage for 5 hours at -70°C (instead of 2 hours at room temperature) the title product of that step is similarly obtained.

Example 8

Ethyl 3R,5S-dihydroxy-6-trityloxyhexanoate

To a vessel containing, at R.T. under an argon atmosphere, 180 ml of dry THF and 45 ml of methanol (dried over molecular seives) is added 45 ml of 1M triethyl borane in THF (45 mmol). The resulting mixture is stirred for 15 minutes at R.T., then cooled to -70°, stirred at -70° for 20 minutes and over a period of 30 minutes 15.20g (35 mmol) of ethyl 5S-hydroxy-6-trityloxy-3-oxo-hexanoate* in 60 ml of dry THF and 15 ml of dry methanol is added with stirring, while the reaction mixture being maintained at -70° to -67°. After the addition is completed the reaction mixture is stirred for 25 minutes at -70°.

1.47g of sodium borohydride (pellets) is then added to the reaction mixture, being maintained at -70° with stirring. About 3 minutes after addition is completed, bubbling is observed and stirring of the mixture is continued for 3 hours at -70°.

The reaction mixture is then quenched by addition of saturated aqueous ammonium chloride at -70° to -60°.

* obtainable from Step 5 Example 1.

200 ml of ethyl acetate is then added and the mixture allowed to slowly warm to R.T. The organic layer is then recovered. The aqueous layer is extracted with 250 ml of ethyl acetate. The combined ethyl acetate extracts are dried, filtered and evaporated to dryness to obtain a residue (the boronate complex).

HPLC grade methanol is then added to the residue and the resulting mixture azeotroped until the boronate complex is entirely converted to the title diol product (as indicated by TLC); additional methanol being added as needed. The title diol product is then recovered by silica column chromatography (eluting with ethyl acetate/hexane; 1:6).

Example 9

Repeating the procedure of Example 8 , but starting with 240 ml of dry THF, 60 ml of dry methanol, 15.20g of ethyl 5S-hydroxy-6-trityloxy-3-oxo-hexanoate and 45 ml of 1M triethylborane (in THF) together in a vessel at about -70°, so that the room temperature stage is omitted, comparable results are obtained.

Example 10

Repeating the procedures of Examples 8 and 9 employing in place of the keto-hydroxy ethyl ester, an equivalent amount of the t.-butyl, methyl or allyl ester analog, comparable results are obtained.

What is claimed is:

1. A process for the preparation of a compound of formula C in 3R,5S-$OP_1$ form:

C 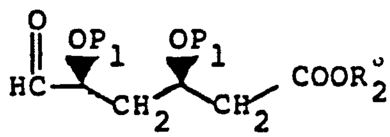

in which $P_1$ is a protective group and $R_2^{\circ}$ is a radical forming an ester inert under the reaction conditions;

which comprises:

1) reacting a carboxylic acid of formula H:

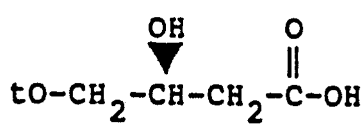

in which t is (triphenyl)methyl; with a bis-magnesium salt of formula Z:

Z $Mg(OOCCH_2COOR^{\circ}_2)_2$

in which $R_2^{\circ}$ is as defined; to obtain a corresponding keto-hydroxy ester of the formula G:

G 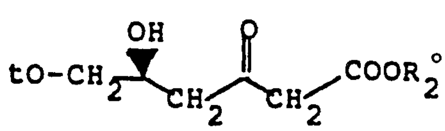

in which t and $R_2^{\circ}$ are as defined;

2) reducing said compound of formula Ga to obtain the corresponding diol of formula F:

F

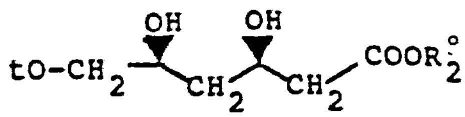

in which t and $R_2^{\circ}$ are as defined:

3) diprotecting said compound of formula F to obtain the corresponding compound of formula E:

E

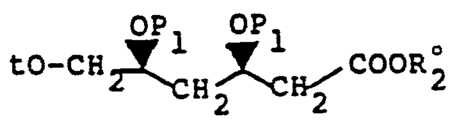

in which $P_1$ and $R_2^{\circ}$ are as defined.

4) cleaving the t-(triphenyl)methoxy radical of said compound of formula E to obtain the corresponding 5-hydroxy-compound of formula D:

D

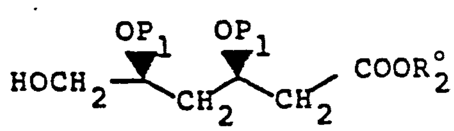

in which $P_1$ and $R_2°$ are as defined; and

5) oxidizing said compound of formula D to obtain the corresponding compound of formula C.

2. A method for preparing a compound of formula I in 3R,5S-form

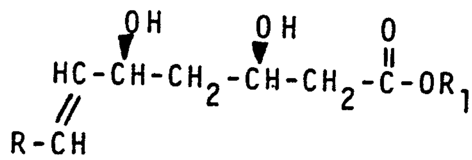

wherein R and $R_1$ are as hereinbefore defined which comprises

a) submitting a compound of formula C as defined in claim 1 to a Wittig reaction with an appropriate R bearing reagent to produce a compound of formula Y

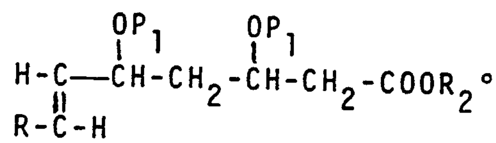

b) deprotecting this compound to produce a compound of formula $I_1$

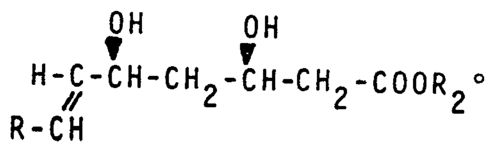

c) if desired or as appropriate converting this in conventional matter into an or into another compound of formula I wherein $R_2°$ is a or another group as $R_1$.

3. A process according to claim 2 wherein the R bearing agent is selected from those of formula Q

$$R\text{-}CH{=}P(Rw)_3$$

or of formula Qy

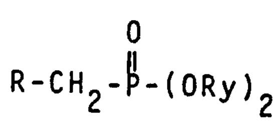

or of formula W

$$R-CH_2-\underset{\underset{O}{\downarrow}}{P}-(OR_7)_2$$

wherein

R is as defined as above Rw is aryl especially phenyl which is unsubstituted or substituted by one or two lower alkyl ($C_{1-4}$) or chloro substituents, n-($C_{1-4}$) alkyl or cyclohexyl; Ry is n-($C_{1-4}$) alkyl especially ethyl; $R_7$ is n-($C_{1-4}$) alkyl or phenyl especially methyl or ethyl.

4. A process according to claim 2 or 3 wherein $R_2°$ is allyl.

5. A process according to claim 2, 3 or 4 wherein the R bearing agent is of formula W as defined in claim 3.

6. A process according to claim 1 wherein the compound of formula H is prepared by a) reducing a di-ester of malic acid having the formula L

$$R_3'OOC-\overset{\overset{OH}{\blacktriangledown}}{C}H-CH_2-COOR_3' \quad L$$

wherein $R_3'$is a radical forming an ester inert under the reaction conditions to form a compound of formula K

$$HOCH_2-\overset{\overset{OH}{\blacktriangledown}}{C}-CH_2-COOR_3' \quad K$$

b) tritylating a compound of formula K to produce a compound of formula J

$$tO-CH_2-\overset{\overset{OH}{\blacktriangledown}}{C}H-CH_2-COOR_3' \quad J$$

and c) saponifying a compound of formula J and neutralising the resulting salt.

7. A compound of formula Y'

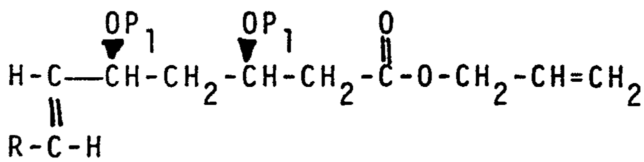

wherein $P_1$ and R are as defined above

8. A compound of Formula I as defined in claim 2 in 3R,5S formwhenever prepared by a method other than resolution of its racemate.

9. A compound of formula I as defined in claim 2 in 3R,5S optical isomer content approaching 100%.

10. Substantially pure (E) erythro-7-[1'-(4"-fluorophenyl)-4'-isopropyl-2'-phenyl-1H-imidazol-S-yl]-3R,5S-dihydroxyhept-6-enoic acid in free from or in the form of its sodium salt or its allyl, ethyl, methyl or t-butyl ester.